# EUROPEAN PATENT APPLICATION

(11) **EP 0 796 997 A1**
(43) Date of publication of application: **24.09.1997**
(21) Application number: 97104548.9
(22) Date of filing: 17.03.1997
(51) Int. Cl.: F04B 43/12, A61M 1/16

(54) **Dialysis Unit**

(30) Priority: 18.03.1996 IT BO960149
(71) Applicant: BELLCO S.p.A., I-41037 Mirandola (IT)
(72) Inventor: Cianciavicchia, Domenico, 64040 Cavuccio (IT)
(74) Representative: Cerbaro, Elena

(57) **Abstract**

The unit includes a dialyzer (3) with two channels, one for the blood and the other for a dialysis fluid; a first conduit (5) along which the dialysis fluid is fed by pumping means to the dialyzer; a second conduit (17) along which the fluid from the dialyzer flows into a first vessel (18); a third conduit (25) connecting a first point (5c) of the first conduit to a second point (17a) of the second conduit; intercepting means installed along the third conduit, along the first conduit downstream from the first point, and along the second conduit upstream from the second point; an assembly (41) installed along the second conduit, between a third (17b) and fourth (17c) point downstream from the second point, and which permits free flow at one stage, and by means of a pump (31) at a different stage; means (23) for determining the flow rate of the fluid fed into the first vessel; a second vessel (71); a fourth conduit (72) connected to the second conduit, between the second and third points, and which terminates in the second vessel; a pump (73) installed along the fourth conduit; and means (74) for determining the flow rate of the fluid fed into the second vessel.

## Description

The present invention relates to a dialysis unit.

The present invention also relates to a dialysis method.

As is known, one of the main problems of current dialysis methods is in determining the weight loss of the patient during treatment. More specifically, currently used units implementing known methods feature a number of peristaltic pumps for pumping fluids along the various conduits of the unit, and sophisticated, high-cost devices for determining the weight loss of the patient by measuring fluid flow along the conduits. As fluid flow is measured using invasive members, i.e. members coming into contact with the fluid circulating along the conduits, the members must be variously sterilized between one treatment and the next. If the peristaltic pumps were accurate enough, however, the flow measuring members could be dispensed with, and the weight loss of the patient determined by simply determining the functional characteristics of the pumps and so working out the resulting fluid flow. Unfortunately, however, the accuracy of peristaltic pumps falls far short of that required for dialysis treatment, which, to prevent cardiac imbalance, requires close monitoring of the weight loss of the patient throughout the treatment.

It is an object of the present invention to provide a dialysis unit designed to overcome the aforementioned drawbacks, i.e. which provides for highly accurate control of the weight loss of the patient.

It is a further object of the present invention to provide a dialysis method implemented by the aforementioned unit.

According to the present invention, there is provided a dialysis unit comprising:
a dialyzer in which a membrane defines two channels, a first for extracorporeal blood, and a second for a dialysis fluid;
a first conduit along which said dialysis fluid flows to said second channel of said dialyzer;
first pumping means for controlling the flow of said dialysis fluid along said first conduit;
a first vessel for receiving part of the fluid from said second channel of said dialyzer;
a second conduit along which part of said fluid from said dialyzer flows to said first vessel; and
sensor means for determining the composition of said dialysis fluid flowing along said first conduit;
   characterized by comprising:
   a third conduit for hydraulically connecting a first point of said first conduit upstream from said dialyzer to a second point of said second conduit downstream from said dialyzer;
   first intercepting means installed along said third conduit, along said first conduit downstream from said first point, and along said second conduit upstream from said second point;
   an assembly installed along said second conduit, between a third point downstream from said second point, and a successive fourth point, and for permitting flow between said third and fourth points freely at one stage, and via second pumping means at another stage;
   means fitted to said first vessel to determine the flow rate of said fluid fed from said second conduit into said first vessel;
   a second vessel;
   a fourth conduit connected to said second conduit at a fifth point between said second and third points, and which terminates in said second vessel;
   third pumping means installed along said fourth conduit; and
   means fitted to said second vessel to determine the flow rate of the fluid fed from said fourth conduit into said second vessel.

According to the present invention, there is also provided a dialysis method, characterized by comprising:
a first cycle comprising enabling hydraulic communication between said first and second conduits via said third conduit; hydraulically isolating said dialyzer, i.e. disabling hydraulic communication downstream from said first point and hydraulic communication upstream from said second point; enabling free hydraulic communication between said third and fourth points; cutting off flow along said fourth conduit; controlling said first pumping means to generate a flow of said dialysis fluid along said first, third and second conduits to said first vessel; determining the flow rate of said fluid fed into said first vessel; and regulating control of said first pumping means to achieve a predetermined flow rate of said fluid fed into said first vessel; and
a second cycle comprising cutting off flow to said third conduit; enabling hydraulic communication between said first and second conduits and said dialyzer; controlling said first pumping means to the same parameters which, in said first cycle, determined a predetermined flow rate of said fluid fed into said first vessel; controlling hydraulic communication between said third and fourth points exclusively by means of said second pumping means to achieve a flow rate into said first vessel equal to that controlled along said first conduit by said first pumping means; and enabling operation of said third pumping means to achieve a flow rate of the fluid fed into said second vessel equal to a predetermined rate proportional to the weight loss of the patient.

A preferred, non-limiting embodiment of the present invention will be described by way of example with reference to the accompanying drawings, in which:
Figure 1 shows a block diagram of a dialysis unit in accordance with the teachings of the present invention;
Figure 2 shows a member forming part of the Figure 1 unit;
Figures 3 and 4 show a different embodiment of the Figure 2 member in two different operating modes.

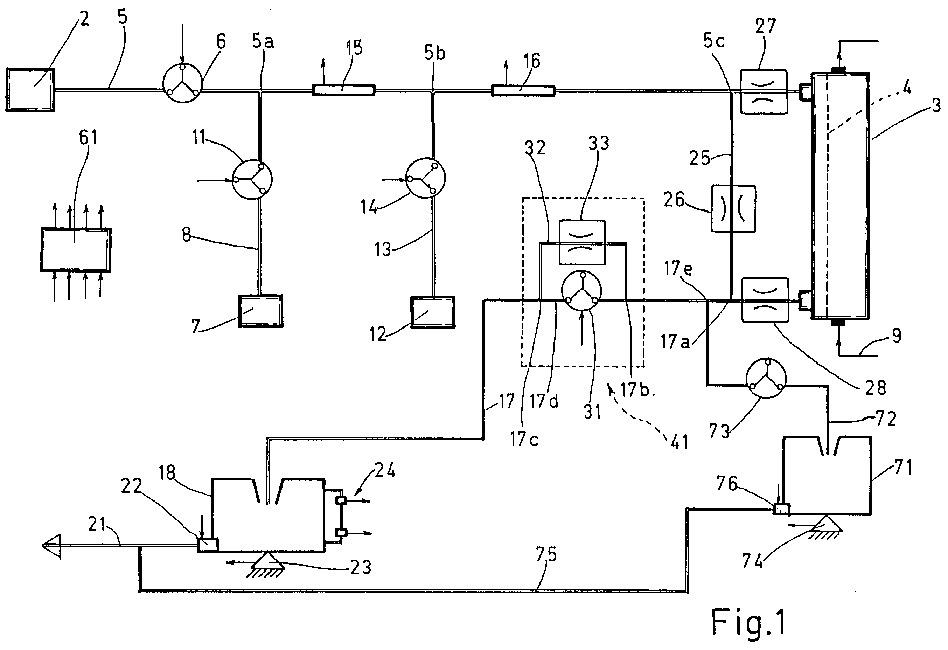

Number 1 in Figure 1 indicates a dialysis unit, which is substantially defined by three parts: a first part in which the dialysis solution is prepared; a second part in which the dialysis solution is used; and a third part for draining the fluid from the second part and controlling the weight loss of the patient.

The second part of unit 1 comprises a known dialyzer 3 in which a membrane 4 defines two channels, a first for extracorporeal blood flowing along a conduit 9, and a second for the dialysis fluid.

The first part of unit 1 comprises:
a water source 2;
a conduit 5 having a first end connected to source 2, and a second end connected to the second channel of dialyzer 3 and from which the dialysis fluid is released;
a known peristaltic pump 6 along conduit 5;
a salt concentrate and water solution source 7;
a conduit 8 hydraulically connecting source 7 to a mixing point 5a defined along conduit 5, downstream from pump 6;
a known peristaltic pump 11 along conduit 8;
a salt concentrate and water solution source 12;
a conduit 13 hydraulically connecting source 12 to a mixing point 5b defined along conduit 5, downstream from point 5a;
a known peristaltic pump 14 along conduit 13;
a preferably conductometric sensor 15 for determining the composition of the fluid fed along conduit 5 between mixing points 5a and 5b; and
a preferably conductometric sensor 16 for determining the composition of the fluid fed along conduit 5 downstream from mixing point 5b.

The third part of unit 1 comprises:
a conduit 17 connected at a first end to the second channel of dialyzer 3;
a vessel 18 housing the second end of conduit 17;
a conduit 21 extending from an outlet formed in vessel 18 to a drain (not shown);
a solenoid valve 22 fitted to conduit 21 at said outlet of vessel 18;
electronic weighing means 23 for determining the weight of vessel 18 and its contents;
electronic means 24 for determining the level of the contents of vessel 18;
a conduit 25 hydraulically connecting a point 5c, defined downstream from sensor 16 along conduit 5, to a point 17a along conduit 17;
fluid intercepting means 26 along conduit 25;
fluid intercepting means 27 along conduit 5, downstream from point 5c;
fluid intercepting means 28 along conduit 17, upstream from point 17a;
a peristaltic pump 31 along conduit 17, downstream from point 17a, and more specifically between two points 17b and 17c between which is defined a branch 17d of conduit 17;
a conduit 32 parallel to branch 17d and therefore hydraulically connecting points 17b and 17c of conduit 17;
fluid intercepting means 33 along conduit 32;
a vessel 71;
a conduit 72 connected to a point 17e, between points 17a and 17b, along conduit 17, and terminating in vessel 71;
a peristaltic pump 73 along conduit 72;
electronic weighing means 74 for weighing vessel 71 and its contents;
a conduit 75 extending from an outlet formed in vessel 71 to said drain (not shown); and
a solenoid valve 76 fitted to conduit 75 at said outlet of vessel 71.

Intercepting means 26, 27, 28 and 33 may be controlled manually and comprise, for example, straightforward grippers, or may be controlled electrically and comprise, for example, solenoid valves. Pump 31, branch 17d, conduit 32 and intercepting means 33 define an assembly 41, which provides for two different operating modes. More specifically, assembly 41 may permit flow along conduit 32 only, by cutting off branch 17d by means of pump 31 (as explained in detail later on), or may permit flow along branch 17d only by operating pump 31 and cutting off conduit 32.

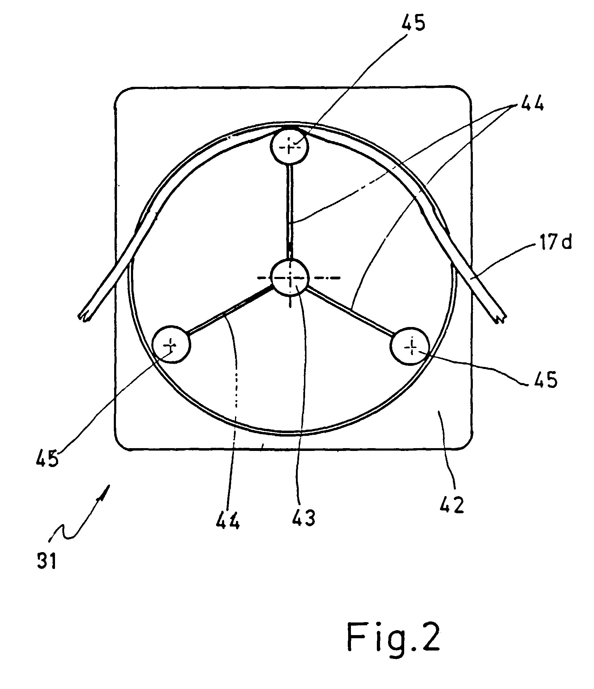

Pump 31 of unit 1 in Figure 1 is a typical peristaltic pump and, as shown schematically in Figure 2, comprises an elongated hollow cylindrical body 42; a rotary shaft 43 rotated by an electric motor (not shown); three arms 44 extending radially from shaft 43 and spaced 120° apart; and idle wheels 45, each fitted to the free end of a respective arm 44. Shaft 43 is coaxial with body 42, which houses arms 44; the length of arms 44 and the diameter of wheels 45 are such that wheels 45 substantially contact the inner surface of body 42; and a portion of the inner surface of body 42 extending about an arc of over 120° houses a portion of conduit 17, so that, when pump 31 is turned off, at least one of wheels 45 presses on said conduit portion, cutting off all flow along conduit 17. As shaft 43 rotates, wheels 45 naturally exert pressure successively along the whole of said portion of conduit 17, thus producing the known pumping effect. Pumps 6, 11, 14 and 73 are identical to pump 31.

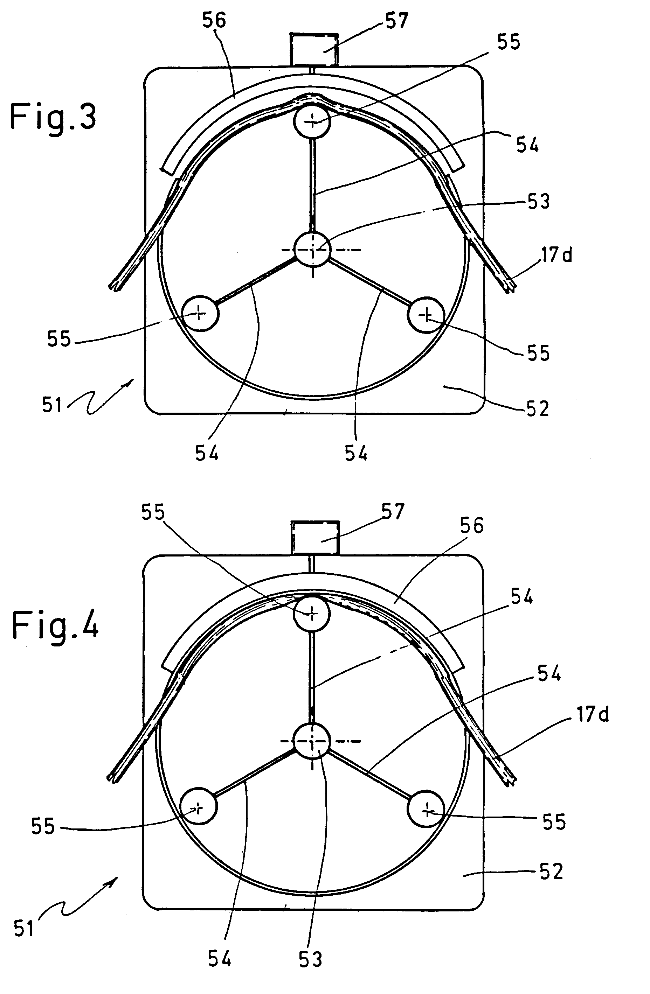

Number 51 in Figures 3 and 4 indicates a known pump, which may be fitted between points 17b and 17c of conduit 17 in place of assembly 41, and which, like assembly 41, provides for two different operating modes. More specifically, pump 51 is designed to permit free flow along branch 17d when disabled, and controlled flow along branch 17d when operated as a normal peristaltic pump. Pump 51 comprises an elongated body 52 formed from a cylindrical body (similar to body 42) from which is removed an axial portion extending in section about an angle of over 120°; a rotary shaft 53 rotated by an electric motor (not shown); three arms 54 extending radially from shaft 53 and spaced 120° apart; and idle wheels 55, each fitted to the free end of a respective arm 54. Shaft 53 is coaxial with body 52, which houses arms 54; and the length of arms 54 and the diameter of wheels 55 are such that wheels 55 substantially contact the inner surface of body 52. Pump 51 also comprises an elongated plate 56 extending in section about an arc of a radius equal to the inside radius of body 52. That is, plate 56 is so formed as to substantially define said portion removed from the cylindrical body from which body 52 is formed, and is movable, via actuating means 57, between a first position in which the inner surface of plate 56 is a continuation of the inner surface of body 52, and a second position in which the distance between the inner surface of plate 56 and the longitudinal axis of body 52 is greater than the inside radius of body 52. In said first position, the inner surface of plate 56 houses a portion of conduit 17, so that, when pump 51 is turned off, at least one of wheels 55 presses on said conduit portion, cutting off all flow along conduit 17. As shaft 53 rotates, wheels 55 naturally exert pressure successively along the whole of said portion of conduit 17, thus producing the known pumping effect. When plate 56 is set to said second position, free flow is permitted along conduit 17.

With reference to Figure 1, unit 1 also comprises an electronic central control unit 61 for controlling operation of unit 1, and more specifically all the peristaltic pumps, solenoid valves 22 and 76, and the intercepting means if these are electrically operated, and which is connected to sensors 15, 16 and means 23, 24, 74.

The dialysis method substantially comprises two main operating cycles, the first of which provides for self-calibrating the flow of dialysis fluid along conduit 5 downstream from point 5b, and the second for determining and controlling the quantity of substances extracted from the blood at dialyzer 3, and hence the weight loss of the patient.

The first cycle comprises:
hydraulically isolating dialyzer 3, i.e. disabling hydraulic communication between dialyzer 3 and the rest of unit 1 via intercepting means 27 and 28;
allowing the dialysis solution to flow along conduit 25 via intercepting means 26;
enabling free flow of the dialysis solution between points 17b and 17c with the pump disabled; that is, enabling free flow along conduit 32 and arresting pump 31 to close branch 17d, when using assembly 41, and maintaining plate 56 in said second position, when using pump 51;
activating solenoid valves 22 and 76 to cut off flow along conduits 21 and 75;
disabling pump 73 to cut off flow along conduit 72;
enabling pumps 6, 11 and 14;
determining the composition of the dialysis solution in known manner by means of sensors 15 and 16;
determining, via weighing means 23, the flow rate of dialysis solution along conduit 5, downstream from point 5b, which is equal to the flow of dialysis solution along conduit 17, downstream from point 17a; and
adjusting control of pumps 6, 11 and 14 to achieve the predetermined flow and composition of dialysis solution, and memorizing the control parameters of pumps 6, 11 and 14 by which said predetermined flow and composition are determined.

The second cycle comprises:
cutting off all flow to conduit 25;
enabling hydraulic communication to and from dialyzer 3, i.e. enabling flow along conduit 5, downstream from point 5c, and along conduit 17, upstream from point 17a;
enabling operation of pumps 6, 11 and 14 according to the memorized control parameters;
peristaltic pump controlling flow between points 17b and 17c; that is, closing conduit 32 and enabling pump 31, when using assembly 41, and moving plate 56 into said first position and enabling pump 51, when using pump 51;
adjusting the control parameters of pump 31 or 51 until means 23 determine a flow rate between points 17b and 17c equal to the predetermined flow rate calibrated in the first cycle; and
operating pump 73 to achieve a predetermined flow rate along conduit 72, as detected by means 74 and equal to a predetermined weight loss of the patient.

Means 24 may be used in place of or in conjunction with means 23, so that flow into vessel 18 may be determined either by weighing (means 23) and/or by determining the level of the fluid fed in time into vessel 18. Means 24 may also be used to determine when a predetermined threshold level is exceeded, in which case, the fluid in vessel 18 is drained off by solenoid valve 22. Self-calibration of the pumps and control of the weight loss of the patient as described above may be performed repeatedly during treatment to control the weight loss of the patient more accurately and possibly also to adjust at various times during treatment both the dialysis solution flow and composition parameters and the desired weight loss.

The advantages of the present invention will be clear from the foregoing description.

In particular, it provides for a unit and method enabling highly accurate control of the weight loss of the patient, by virtue of control not requiring detection of the functional characteristics of the peristaltic pumps to determine the flow rates involved. Moreover, the method is fast and easy to implement, and may be performed repeatedly during treatment. And finally, the unit itself is straightforward in design, and requires no invasive flow detecting devices, and hence, no sterilization between one treatment and the next.

Clearly, changes may be made to unit 1 and the method as described and illustrated herein without, however, departing from the scope of the present invention.

## Claims

1. A dialysis unit comprising:
a dialyzer (3) in which a membrane (4) defines two channels, a first for extracorporeal blood, and a second for a dialysis fluid;
a first conduit (5) along which said dialysis fluid flows to said second channel of said dialyzer (3);
first pumping means (6, 11, 12) for controlling the flow of said dialysis fluid along said first conduit (5);
a first vessel (18) for receiving part of the fluid from said second channel of said dialyzer (3);
a second conduit (17) along which part of said fluid from said dialyzer (3) flows to said first vessel (18); and
sensor means (15, 16) for determining the composition of said dialysis fluid flowing along said first conduit (5);
characterized by comprising:
a third conduit (25) for hydraulically connecting a first point (5c) of said first conduit (5) upstream from said dialyzer (3) to a second point (17a) of said second conduit (17) downstream from said dialyzer (3);
first intercepting means (26, 27, 28) installed along said third conduit (25), along said first conduit (5) downstream from said first point (5c), and along said second conduit (17) upstream from said second point (17a);
an assembly (41, 51) installed along said second conduit (17), between a third point (17b) downstream from said second point (17a), and a successive fourth point (17c), and for permitting flow between said third (17b) and fourth (17c) points freely at one stage, and via second pumping means (31, 51) at another stage;
means (23, 24) fitted to said first vessel (18) to determine the flow rate of said fluid fed from said second conduit (17) into said first vessel (18);
a second vessel (71);
a fourth conduit (72) connected to said second conduit (17) at a fifth point (17e) between said second (17a) and third (17b) points, and which terminates in said second vessel (71);
third pumping means (73) installed along said fourth conduit (72); and
means (74) fitted to said second vessel (71) to determine the flow rate of the fluid fed from said fourth conduit (72) into said second vessel (71).

2. A unit as claimed in Claim 1, characterized in that, between said third (17b) and fourth (17c) points, said assembly (41) comprises a first branch (17d) along which are installed said second pumping means defined by a first peristaltic pump (31), which, when disabled, cuts off flow along said first branch (17d), and, when enabled, generates a pumping effect along said first branch (17d); and a second branch (32) parallel to the first, and along which are installed second intercepting means (33).

3. A unit as claimed in Claim 1, characterized in that said assembly comprises a peristaltic pump (51), which, when disabled, permits free flow along said second conduit (17), and, when enabled, generates a pumping effect along said second conduit (17).

4. A unit as claimed in Claim 2 or 3, characterized by comprising a first source (2) of water from which said first conduit (5) originates; a second source (7) of a salt concentrate and water solution from which originates a fifth conduit (8) connected to a fifth point (5a) of said first conduit (5); and a third source (12) of a salt concentrate and water solution from which originates a sixth conduit (13) connected to a sixth point (5b) of said first conduit (5); said first pumping means comprising a second peristaltic pump (6) installed along said first conduit (5) upstream from said fifth (5a) and sixth (5b) points, a third peristaltic pump (11) installed along said fifth conduit (8), and a fourth peristaltic pump (14) installed along said sixth conduit (13); and said third pumping means comprising a fifth peristaltic pump installed along said fourth conduit (72).

5. A unit as claimed in any one of the foregoing Claims, characterized in that said means for determining the flow rate of said fluid fed into said first vessel (18) comprise a weighing device (23) for weighing said first vessel (18).

6. A unit as claimed in any one of the foregoing Claims, characterized in that said means for determining the flow rate of said fluid fed into said first vessel (18) comprise a detecting device (24) for detecting the level of said fluid in said first vessel (18).

7. A unit as claimed in any one of the foregoing Claims, characterized in that said means for determining the flow rate of said fluid fed into said second vessel (71) comprise a weighing device (74) for weighing said second vessel (71).

8. A unit as claimed in any one of the foregoing Claims, characterized by comprising an electronic central control unit (61) for controlling said first (6, 11, 14), second (31, 51) and third (73) pumping means, and to which are connected said sensor means (15, 16) and said means (23, 24, 74) for determining the flow rate of the fluid fed into said vessels (18, 71).

9. A dialysis method as claimed in any one of the foregoing Claims, characterized by comprising:
a first cycle comprising enabling hydraulic communication between said first (5) and second (17) conduits via said third conduit (23); hydraulically isolating said dialyzer (3), i.e. disabling hydraulic communication downstream from said first point (5c) and hydraulic communication upstream from said second point (17a); enabling free hydraulic communication between said third (17b) and fourth (17c) points; cutting off flow along said fourth conduit (72); controlling said first pumping means (6, 11, 14) to generate a flow of said dialysis fluid along said first (5), third (25) and second (17) conduits to said first vessel (18); determining the flow rate of said fluid fed into said first vessel (18); and regulating control of said first pumping means (6, 11, 14) to achieve a predetermined flow rate of said fluid fed into said first vessel (18); and
a second cycle comprising cutting off flow to said third conduit (25); enabling hydraulic communication between said first (5) and second (17) conduits and said dialyser (3); controlling said first pumping means (6, 11, 14) to the same parameters which, in said first cycle, determined a predetermined flow rate of said fluid fed into said first vessel (18); controlling hydraulic communication between said third (17b) and fourth (17c) points exclusively by means of said second pumping means (31, 51) to achieve a flow rate into said first vessel (18) equal to that controlled along said first conduit (5) by said first pumping means (6, 11, 14); and enabling operation of said third pumping means (73) to achieve a flow rate of the fluid fed into said second vessel (71) equal to a predetermined rate proportional to the weight loss of the patient.

10. A method as claimed in Claim 9 dependent on Claim 4, characterized in that, in said first cycle, the control parameters of said first pumping means are varied to achieve both said predetermined flow rate and a predetermined composition of said dialysis fluid.

11. A method as claimed in Claim 9 and/or 10, characterized in that, in said cycles, the flow rate of said fluid fed into said vessels (18, 71) is determined by weighing the vessels (18, 71).

12. A method as claimed in Claim 9 and/or 10, characterized in that, in said cycles, the flow rate of said fluid fed into said first vessel (18) is determined by determining the level of said fluid in said first vessel (18).

13. A method as claimed in any one of the foregoing Claims from 9 to 12, characterized in that, in said first cycle, the control parameters of said first pumping means whereby said predetermined flow rate of said dialysis fluid is achieved are memorized.
